Europäisches Patentamt

European Patent Office

Office européen des brevets

(19)

(11) Publication number: **0 203 632 B1**

# EUROPEAN PATENT SPECIFICATION

(45) Date of publication of patent specification: **07.08.91**

(51) Int. Cl.5: **B01J 29/04, C07C 37/08, C07C 39/04, C07C 49/08, C07C 45/53**

(21) Application number: **86200664.0**

(22) Date of filing: **18.04.86**

(54) Catalyst for the selective decomposition of cumene hydroperoxide and process for preparing it.

(30) Priority: **23.04.85 IT 2045885**

(43) Date of publication of application:
**03.12.86 Bulletin 86/49**

(45) Publication of the grant of the patent:
**07.08.91 Bulletin 91/32**

(84) Designated Contracting States:
**AT BE CH DE FR GB LI LU NL SE**

(56) References cited:
**EP-A- 0 113 473**
**EP-A- 0 125 065**
**EP-A- 0 168 978**
**GB-A- 2 024 790**
**US-A- 4 254 297**

(73) Proprietor: **ENICHEM SYNTHESIS S.p.A.**
**Via Ruggero Settimo 55**
**I-90139 Palermo(IT)**

(72) Inventor: **Romano, Ugo**
**Via XXV Aprile 10**
**I-20059 Vimercate Milan(IT)**
Inventor: **Clerici, Mario Gabriele**
**Via Europa 34**
**I-20097 San Donato Milanese Milan(IT)**
Inventor: **Bellussi, Giuseppe**
**Via Alberto Scopo 44**
**I-29100 Piacenza(IT)**
Inventor: **Buonomo, Franco**
**Via Trento 4**
**I-20097 San Donato Milanese Milan(IT)**

(74) Representative: **Roggero, Sergio et al**
**Ing. Barzanò & Zanardo Milano S.p.A. Via Borgonuovo 10**
**I-20121 Milano(IT)**

## Description

This invention relates to a catalyst for the selective decomposition of cumene hydroperoxide into phenol and acetone, and to the use of said catalyst for that decomposition reaction.

Conventionally, cumene is peroxidated to cumene hydroperoxide, and the latter is decomposed by the agency of an acidic catalyst, which is sulphuric acid: such a procedure produces phenol and acetone, but accompanied by acetophenone and sulphonated by-products which pollute the desired products and are difficult to separate.

The present invention aims at carrying out the decomposition reaction in question with the aid of a catalyst other than sulphuric acid, which is a modified zeolite. EP-A-168 978 discloses zeolites which contain oxides of silicon, aluminium and boron: these zeolites can be used, among others, for preparing the modified zeolites to be used according to the present invention.

The invention, therefore, provides a silicon-, aluminium- and boron-based catalyst for the selective decomposition of cumene hydroperoxide into phenol and acetone, in which silicon-, aluminium- and boron oxides form crystals having a zeolite-structure, and aluminium and boron partially replace silicon in the crystalline structure of silica, characterized in that said crystals are interconnected by oligomeric silica. It is preferred that the molar ratio of the silica of the zeolite crystals to the oligomeric silica is from 80 to 95.

According to another aspect of this invention, the catalyst is one having the form of microspheres of a size of from 5 $\mu$m to 100 $\mu$m, and, preferably, from 20 $\mu$m to 100 $\mu$m.

According to a particular aspect of the invention, after calcination at 550°C and cooling to room temperature, the catalyst has the formula:

0,017 to 0,0025 $Al_2O_3$ . 0,1 to 0,005 $B_2O_3$ - $SiO_2$ with from 0,2 to 0,5 **water**.

The invention, furthermore, provides a process for preparing the catalyst defined in claim 1, comprising:

a) a first step of preparation of zeolite crystals in which aluminium and boron replace silicon in the crystalline structure of the silica, and

b) a second step in which the as-obtained zeolite crystals are dispersed in a clear solution obtained by hydrolyzing a tetraalkyl silicate in an aqueous solution of tetraalkylammonium hydroxide for a time of from 1 to 2 hours at a temperature of from 60°C to 70°C, whereafter the resultant suspension is atomized to prepare microspheres having an average diameter of from 5 $\mu$m to 100 $\mu$m, and said microspheres are calcined at a temperature of from 500°C to 600°C for a time from 2 h to 10 h.

The first step is accomplished by starting from alkyl silicates (alkyl groups of from 1 to 4 carbon atoms), in particular from tetramethyl- and/or tetraethylsilicate, possibly in aqueous solution which are added to an alcoholic solution (aliphatic alcohol of from 1 to 6 carbon atoms) of an aluminium salt at a concentration of preferably from 4 to 5%, preferably an alcoholic solution of Al nitrate or acetate, the whole being then mixed with an aqueous solution of tetracrocylammonium hydroxide and $H_3BO_3$ . From the reaction medium foreign cations, in particular cations of alkaline or alkaline-earth metals, must be absent.

The whole is heated under stirring to a temperature comprised within the range of from 50 to 80°C, preferably to 60°C, to the purpose of hydrolyzing the alkyl silicate and removing an amount as high as possible of the alkyl alcohol obtained from the hydrolysis and of the alcohol outcoming from the solution of the aluminium salt.

After the hydrolysis of the alkyl silicate and the removal of the alcohols, the resulting residual solution is charged into an autoclave equipped with a stirrer, and is heated, under its autogenous pressure, at a temperature of 150 to 250°C, for a time period comprised within the range of from 3 hours to 10 days, a suspension of crystals being obtained. The crystals of the suspension are then separated from the mother liquors, preferably by centrifuging, washed in deionized water and dried, preferably by centrifuging.

The second step consists in dispersing the above said zeolite crystals in a clear solution obtained by hydrolyzing a tetraalkyl silicate (alkyl group of from 1 to 4 carbon atoms), preferably tetramethyl- or tetraethyl-silicate, in an aqueous solution of tetraalkylammonium (TAA) hydroxide (alkyl group of from 1 to 5 carbon atoms) for a time comprised within the range of from 1 to 2 hours at a temperature of 60-70°C.

The suspension obtained is atomized, preferably by means of a spray drier, microspheres having an average diameter comprised within the range of from 5 to 1000 $\mu$m, preferably of from 20 to 100 $\mu$m, being obtained.

The microspheres are then calcined at a temperature of from 500 to 600°C, preferably at 550°C for 2-10 hours, preferably for 5 hours.

The reaction mixture for the synthesis of the zeolite has the following molar composition:

0.1-0.35 tetrapropylammonium oxide; $SiO_2$;

0.0025-0.01 $Al_2O_3$; 0.2-0.02 $B_2O_3$; 20-40 $H_2O$.

The reaction mixture for the preparation of the catalyst has the following composition by mol (with the

2

exclusion of $Al_2O_3$ and $B_2O_3$):

0.05-0.15 tetraalkylammonium oxide; 4-19 $SiO_2$ (of zeolite);

1 $SiO_2$ (of tetraalkyl Silicate); 80-600 $H_2O$.

A third object of the present invention is the method for the selective decomposition of cumene hydroperoxide into phenol and acetone using the above said catalyst.

The method according to the invention consists in contacting cumene hydroperoxide with the catalyst, preferably placed inside a reactor on one or more stationary or fluidified beds at a temperature of from 20 to 120°C, preferably of 40-60°C, and in discharging the reaction products and indecomposed cumene hydroperoxide. Cumene hydroperoxide can be supplied as such, or diluted in a suitable solvent, in particular cumene.

The space speed LHSV referred to cumene hydroperoxide in case of continuous operating is comprised between 0.5 and 10 $h^{-1}$. For the batchwise operation, the permanence time is comprised within the range of from 1' min to 120' min it being preferably of 15 min. - 20 min.

Some Examples having the purpose of better illustrating the invention shall be now supplied.

Example No. 1
- - - - - - - -

An amount of 67.8 g of $Al(NO_3)_3.9H_2O$ is dissolved in 1275 g of ethyl alcohol and to these, under stirring, 2819 g of tetraethyl-silicate is added, with stirring being continued until a homogeneous and clear solution is obtained.

Into a stainless steel vessel 1036 g of deionized water, 8878 g of an aqueous solution at 15.5% by weight of tetrapropyl-ammonium ($TPA^+$) hydroxide and 167.5 g of powder of boric acid are added sequentially and under stirring. When the acid has been completely dissolved, to this solution the previously obtained solution is added, and the mixture is stirred while being heated at 60°C for about 4 hours and in any case until the hydrolysis of the silicate is completed, and the present ethyl alcohol has been nearly completely removed. The molar composition of the reaction mixture is the following:

$SiO_2/Al_2O_3 = 150$; $SiO_2/B_2O_3 = 10$; $TPA^+/SiO_2 = 0.5$;

$H_2O/SiO_2 = 35$.

The so-obtained solution is charged into an autoclave equipped with a stirrer and is heated under its autogenous pressure under stirring for 4 hours at 170°C. The discharged product is centrifuged and the cake is accurately dispersed in 70 litres of deionized water, the obtained suspension is again centrifuged, yielding a washed cake.

An amount of 2451 g of tetraethylsilicate is hydrolyzed in the presence of 216 g of a solution at 15.5% of tetrapropyl-ammonium hydroxide and 1300 g of deionized $H_2O$. The washed cake discharged from the centrifuge is accurately dispersed into the so-obtained clear solution, until a milky suspension is obtained. Such suspension is fed to a spray dryer and atomized so as to obtain microspheres having an average diameter comprised within the range of from 10 to 30 $\mu$m. The product is calcined for 1 h and 30 min. under $N_2$, with the temperature being raised from room temperature to 550°C, and then at a constant temperature of 550°C in air for further 4 hours. The finished catalyst (A) is thus obtained, the chemical analysis of which is as follows:

$SiO_2$: 96.87; $Al_2O_3$: 1.851; $B_2O_3$: 1.029; $H_2O$: balance to 100.

To 50 g of a phenol / acetone (50% by mol) solution, kept at a temperature controlled at 40°C, 3 g of catalyst A and then 17 g of cumene hydroperoxide are added. The decomposition is monitored by iodimetric titration and gas-chromatographic analysis.

The results are reported in Table 1.

Example No. 2
- - - - - - - -

Following the procedure as disclosed in Example 1, the washed cake discharged from the centrifuge is obtained. The solid is calcined under $H_2$ with the temperature being raised from room temperature to 550°C and then at a constant temperature of 550°C in air for 4 hours.

The product obtained (B) has the following chemical composition:

$SiO_2$: 96.93; $Al_2O_3$: 1.968; $B_2O_3$: 0.852; $H_2O$: balance to 100.

By the reactants and procedures as disclosed in Example 1, with the exception that 3 g of catalyst B is used in lieu of the 3 g of catalyst A, the results described in Table 1 are obtained.

Example No. 3 (comparison)
- - - - - - - - - - - - - - -

Into a 100-cc beaker, 1 g of $Al(NO_3)_3 \cdot 9H_2O$ is dissolved in 18.8 g of anhydrous $C_2H_5OH$ and to the solution obtained, 41.6 g of tetraethyl-silicate is added.

Into another vessel of pyrex glass, 15.3 g of $H_2O$, 131 g of aqueous solution of tetrapropyl-ammonium hydroxide at 15.5% by weight and the previously prepared alcoholic solution are added under stirring. The whole is heated at 60°C under stirring for about 4 hours, and in any case until the hydrolysis of the silicate has ended and ethyl alcohol has been nearly completely removed. The solution obtained, which has the following molar composition:

$SiO_2/Al_2O_3 = 150$; $TPA^+/SiO_2 = 0,5$; $H_2O/SiO_2 = 35$,

is charged into an autoclave equipped with stirrer and heated under stirring, under its autogenous pressure, at 170°C for 4 hours.

The discharged milky suspension is centrifuged, the cake is dispersed in 500 g of deionized water and the suspension is centrifuged. The washed cake is then calcined by increasing the temperature, over a 1h and 30min. time, under $N_2$, from room temperature to 550°C and then at a constant temperature of 550°C in air for 4 hours. The product obtained (C) has the following composition:

$SiO_2$: 96.58; $Al_2O_3$: 1.077; $H_2O$: balance to 100.

By the reactants and procedures as disclosed in Example 1, with the exception that 3 g of catalyst C is used in lieu of the 3 g of catalyst A, the results described in Table 1 are obtained.

Example No. 4 (comparison)

Into a 300-cc beaker of pyrex glass, 131 g of aqueous solution of tetrapropyl-ammonium hydroxide at 15.5% by weight 15.3 of deionized $H_2O$, and 2.5 g of $H_3BO_3$ are added under stirring. The whole is stirred, with heating if necessary, until a clear solution is obtained, then 41.6 g of tetraethyl-silicate are added, always under stirring. The stirring is continued for 4 hours while heating at 60°C, and anyway until the complete hydrolysis of the silicate has occurred, and to the nearly total removal of ethyl alcohol. The solution obtained, which has the following molar composition:

$SiO_2/B_2O_3 = 10$; $TPA^+/SiO_2 = 0,5$; $H_2O/SiO_2 = 35$,

is charged into an autoclave equipped with stirrer and heated under stirring, under its autogenous pressure, at 170°C for 4 hours.

The discharged milky suspension is centrifuged, the cake is dispersed in 500 g of deionized water and the suspension is centrifuged. The washed cake is then calcined by increasing the temperature, over a 1h and 30 min. time, under $N_2$, from room temperature to 550°C and then at a constant temperature of 550°C in air for 4 hours. The produce obtained (D) has the following composition:

$SiO_2$: 97.55; $B_2O_3$: 1.954; $H_2O$: $H_2O$: balance to 100.

By the reactants and procedures as disclosed in Example 1, with the exception that 3 g of catalyst D is used in lieu of the 3 g of catalyst A, the results described in Table 1 are obtained.

Example No. 5

By the procedure and reactants as disclosed in Example 1, the decomposition of cumene hydroperoxide (CHP) is carried out by using, in lieu of the 3 g of catalyst A, 0.2 g of catalyst E (sulphuric acid in aqueous solution at 96%) and adjusting the addition rate of CHP so that the addition takes place within a time of 10'; the temperature is kept always at 40°C. After a total time of 30 min. the reaction mixture is neutralized by sodium bicarbonate, and analyzed by iodimetric titration and gas-chromatographic analysis.

The results are reported in Table 1.

## TABLE 1

| CATA-LYST | REACTION TIME | CHP % CONV. | % SELECTIVITY PHENOL | | % SELECTIVITY ACETOPHENONE | | OTHERS | |
|---|---|---|---|---|---|---|---|---|
| A | 15 min. | 100 | 96 | % | 3 | % | 1 | % |
| B | 20 min. | 100 | 96 | % | 3 | % | 1 | % |
| C | 120 min. | 80 | 85.5 | % | 8 | % | 6.5 | % |
| D | 120 min. | 40 | 80.5 | % | 12.2 | % | 7.8 | % |
| E | 30 min. | 100 | 95 | % | 3 | % | 2 | % |

Example No. 6

An example of continuous synthesis of phenol and acetone is now given.

A test is carried out by operating in continuous by using an equipment consisting of a glass reactor of 0.5 l in volume, equipped with mechanical stirring means, automatic level control, temperature control system, inlet for the cumene hydroperoxide solution, outlet for the outflowing stream, and equipped with a filtering candle to avoid losses of catalyst.

To exemplifying purpose, a test carried out at 60°C, by feeding a solution of CHP (84%) in cumene at the flow rate of 0.1 l/h, with a reactants volume in the reactor of 0,12 l, 7 g of catalyst A, is described. The reaction is monitored by means of iodimetric titration and gaschromatographic analysis of samples drawn at regular time intervals.

The conversion of cumene hydroperoxide, after 10 hours of operation, is of 90%, with the effluent having the following composition (% by weight), with the exclusion of cumene:

| | | |
|---|---|---|
| CHP | 10 | % |
| Phenol | 54.2 | % |
| Acetone | 33.3 | % |
| Acetophenone | 1.3 | % |
| Others | 1.0 | % |

The matter yields referred to CHP are generally higher than 98%.

As regards the catalyst, at the end of the test this results to still have the same initial characteristics, apart from a light brown colour.

It can be totally reactivated either by washing at high temperature (100-150°C) with methanol or with another suitable solvent, or by calcination at 550°C in air.

When it is used again after these treatments, it displays a behaviour identical to that of a sample of just prepared catalyst.

**Claims**

1. Silicon-, aluminium- and boron-based catalyst for the selective decomposition of cumene hydroperoxide into phenol and acetone, in which silicon-, aluminium- and boran oxides form crystals having a zeolite-structure, and aluminium and boron partially replace silicon in the crystalline structure of silica, characterized in that said crystals are interconnected by oligomeric silica.

2. Catalyst according to claim 1, wherein the molar ratio of the silica of the zeolite crystals to the oligomeric silica is from 80 to 95.

3. Catalyst according to claim 1, having the form of microspheres of a size of from 5 $\mu$m to 1000 $\mu$m, preferably from 20 $\mu$m to 100 $\mu$m.

4. Catalyst according to claim 1, wherein, after calcination at 550°C and cooling to room temperature, it has the formula:
0,017 to 0,0025 $Al_2O_3$ . 0,1 to 0,005 $B_2O_3$ . $SiO_2$ with from 0,2 to 0,5 **water**.

5. Process for preparing the catalyst defined in claim 1, comprising:
a) a first step of preparation of zeolite crystals in which aluminium and boron replace silicon in the crystalline structure of the silica, and
b) a second step in which the as-obtained zeolite crystals are dispersed in a clear solution obtained by hydrolyzing a tetraalkyl silicate in an aqueous solution of tetraalkylammonium hydroxide for a time of from 1 to 2 hours at a temperature of from 60°C to 70°C, whereafter the resultant suspension is atomized to prepare microspheres having an average diameter of from 5 $\mu$m to 1000 $\mu$m, and said microspheres are calcined at a temperature of from 500°C to 600°C for a time from 2 h to 10 h.

6. Process according to claim 5, wherein the tetraalkyl silicate has C1-C4 alkyl groups.

7. Process according to claim 6, wherein the tetraalkyl silicate is selected from tetramethyl silicate and tetraethyl silicate.

8. Process according to claim 5, wherein the microspheres are calcined at 550°C.

9. Process according to claim 5, wherein the tetraalkylammonium hydroxide has C1-C5 alkyl groups.

10. Process according to claim 5, wherein, in the first step, zeolite crystals are prepared by adding an alklyl silicate to an alcoholic solution of an aluminium salt, mixing the resulting solution with an aqueous solution of tetrapropyl ammonium hydroxide and $H_3BO_3$, heating under stirring to a temperature from 50° to 80°C to hydrolyze the alkyl silicate and remove the alkyl alcohol deriving both from said hydrolysis and from the alcoholic solution of the aluminium salt, and heating the residual solution to a temperature from 150° to to 250°C in an autoclave, under its autogenous pressure, for a time period of 3 hours to 10 days, to obtain a suspension of crystals.

11. Process according to claim 10, wherein the reactants are mixed according to the following molar composition:
0,1 to 0,35 tetrapropylammonium oxide
$SiO_2$;
0,0025 to 0,01 $Al_2O_3$;
0,2 to 0,02 $B_2O_3$, and
20 to 40 $H_2O$.

12. Process according to claim 5, wherein, in the second step of interconnection of the zeolite crystals by oligomeric silica, the reactants are mixed according to the following molar composition:
0,05 to 0,15 tetraalkylammonium oxide
4 to 19 $SiO_2$ (of the zeolite);
1 $SiO_2$ (of the tetraalkyl silicate), and
80 to 600 $H_2O$.

13. Use of the catalyst defined in claim 1 for the selective decomposition of cumene hydroperoxide into phenol and acetone.

14. Use of the catalyst according to claim 13, wherein cumene hydroperoxide is contacted by the catalyst at a temperature of from 20°C to 120°C, preferably from 40°C to 60°C.

**Revendications**

1. Catalyseur à base de silicium, d'aluminium et de bore pour la décomposition sélective de l'hydroperoxyde de cumène en phénol et acétone, dans lequel les oxydes de silicium, d'aluminium et de bore forment des cristaux ayant une structure zéolithique, et où l'aluminium et le bore remplacent partiellement le silicium dans la structure cristalline de la silice, **caractérisé** en ce que lesdits cristaux sont interconnectés (réticulés) par de la silice oligomère.

2. Catalyseur selon la revendication 1 où le rapport molaire de la silice des cristaux de zéolithe à la silice oligomère est de 80 à 95.

3. Catalyseur selon la revendication 1 ayant la forme de microsphères d'une dimension de 5 $\mu$m à 1000 $\mu$m et de préférence de 20 $\mu$m à 100 $\mu$m.

4. Catalyseur selon la revendication 1, où après calcination à 550°C et refroidissement à la température ambiante, il a la formule :
   0,017 à 0,0025 $Al_2O_3$ . 0,1 à 0,005 $B_2O_3$ . $SiO2$ avec de 0,2 à 0,5 eau.

5. Procédé de préparation du catalyseur défini dans la revendication 1, comprenant :
   (a) une première étape de préparation de cristaux de zéolithe dans lesquels l'aluminium et le bore remplacent le silicium dans la structure cristalline de la silice, et
   (b) une seconde étape dans laquelle les cristaux de zéolithe ainsi obtenus sont dispersés dans une solution claire obtenue par hydrolyse d'un tétraalkylsilicate dans une solution aqueuse d'hydroxyde de tétraalkylammonium, pendant une durée de 1 à 2 heures, à une température de 60°C à 70°C, à la suite de quoi la suspension résultante est atomisée pour préparer des microsphères ayant un diamètre moyen de 5 $\mu$m à 1000 $\mu$m, et lesdits microsphères sont calcinés à une température de 500°C à 600°C pendant une durée de 2 heures à 10 heures.

6. Procédé selon la revendication 5 où le tétraalkylsilicate comporte des groupes alkyle en C1-C4.

7. Procédé selon la revendication 6 où le silicate de tétraalkyle est choisi parmi le tétraméthylsilicate et le tétraéthylsilicate.

8. Procédé selon la revendication 5 où les microsphères sont calcinées à 550°C.

9. Procédé selon la revendication 5 où l'hydroxyde de tétraalkylammonium comporte des groupes alkyle C1-C5.

10. Procédé selon la revendication 5 où dans la première étape les cristaux zéolithes sont préparés en ajoutant un alkylsilicate à une solution alcoolique de sel d'aluminium, en mélangeant la solution résultante avec une solution aqueuse d'hydroxyde de tétrapropylammonium et $H_3BO_3$, en chauffant, sous agitation, à une température de 50 à 80°C pour hydrolyser l'alkylsilicate et éliminer l'alcool alkylique provenant tous deux de ladite hydrolyse et de la solution alcoolique du sel d'aluminium, et en chauffant la solution résiduelle à une température de 150 à 250°C, dans un autoclave, sous pression autogène, pendant une durée de 3 heures à 10 jours, pour obtenir une suspension de cristaux.

11. Procédé selon la revendication 10 où les réactifs sont mélangés selon la composition molaire suivante :
    0,1 à 0,35 oxyde de tétrapropylammonium
    $SiO_2$ ;
    0,0025 à 0,01 $Al_2O_3$ ;
    0,2 à 0,02 $B_2O_3$, et
    20 à 40 $H_2O$.

12. Procédé selon la revendication 5, où dans la seconde étape d'interconnexion des cristaux de zéolithe par de la silice oligomère, on mélange les réactifs selon la composition molaire suivante :
    0,05 à 0,15 oxyde de tétraalkylammonium
    4 à 19 $SiO_2$ (de la zéolithe) ;
    1 $SiO_2$ (du tétraalkylsilicate), et

80 à 600 H$_2$O.

13. Utilisation du catalyseur défini dans la revendication 1 pour la décomposition sélective d'hydroperoxyde de cumène en phénol et acétone.

14. Utilisation du catalyseur selon la revendication 13, où l'hydroperoxyde de cumène est mis en contact par le catalyseur à une température de 20° C à 120° C et de préférence de 40° C à 60° C.

**Patentansprüche**

1. Katalysator auf der Basis von Silizium, Aluminium und Bor für die selektive Zersetzung von Cumolhydroperoxid zu Phenol und Aceton, worin Silizium-, Aluminium- und Boroxide Kristalle mit einer Zeolithstruktur ausbilden und Aluminium und Bor partiell Silizium im Kristallgitter von Siliziumdioxid ersetzen, dadurch gekennzeichnet, daß diese Kristalle durch oligomeres Siliziumdioxid miteinander verbunden sind.

2. Katalysator nach Anspruch 1, worin das Molvehältnis des Siliziumdioxids der Zeolithkristalle zu oligomerem Siliziumdioxid von 80 bis 95 beträgt.

3. Katalysator nach Anspruch 1 in Form von Mikrokügelchen mit einer Größe von 5 $\mu$m bis 1.000 $\mu$m, vorzugsweise von 20 $\mu$m bis 100 $\mu$m.

4. Katalysator nach Anspruch 1, der, nach Calcinierung bei 550° C und Abkühlen auf Raumtemperatur, die Formel:
0,017 bis 0,0025 Al$_2$O$_3$ . 0,1 bis 0,005 B$_2$O$_3$ SiO$_2$ mit 0,2 bis 0,5 Wasser aufweist.

5. Verfahren zur Herstellung des in Anspruch 1 definierten Katalysators, umfassend:
a) eine erste Stufe der Bereitung von Zeolithkristallen, worin Aluminium und Bor Silizium in der Kristallstruktur von Siliziumdioxid ersetzen, und
b) eine zweite Stufe, worin die solcherart erhaltenen Zeolithkristalle in einer klaren Lösung, die durch Hydrolysieren eines Tetraalkylsilikats in einer wäßrigen Tetraalkylammoniumhydroxidlösung während 1 bis 2 Stunden bei einer Temperatur von 60 bis 70° C erhalten worden ist, dispergiert werden, wonach die gebildete Suspension zur Ausbildung von Mikrokügelchen mit einem mittleren Durchmesser von 5 $\mu$m bis 1.000 $\mu$m zerstäubt wird und diese Mikrokügelchen bei einer Temperatur von 500 bis 600° C während 2 bis 10 Stunden calciniert werden.

6. Verfahren nach Anspruch 5, worin das Tetraalkylsilicat C$_1$-bis C$_4$-Alkylgruppen aufweist.

7. Verfahren nach Anspruch 6, worin das Tetraalkylsilicat unter Tetramethylsilicat und Tetraethylsilicat ausgewählt wird.

8. Verfahren nach Anspruch 5, worin die Mikrokügelchen bei 550° C calciniert werden.

9. Verfahren nach Anspruch 5, worin das Tetraalkylammoniumhydroxid C$_1$ - C$_5$-Alkylgruppen aufweist.

10. Verfahren nach Anspruch 5, worin in der ersten Stufe Zeolithkristalle durch Zusetzen eines Alkylsilicats zu einer alkoholischen Lösung eines Aluminiumsalzes und Vermischen der gebildeten Lösung mit einer wäßrigen Lösung von Tetrapropylammoniumhydroxid und H$_3$BO$_3$, Erhitzen unter Rühren auf eine Temperatur von 50 bis 80° C zum Hydrolysieren des Alkylsilicats und Abtrennen des Alkylalkohols, der sowohl aus dieser Hydrolyse herrührt als auch aus der alkoholischen Lösung des Aluminiumsalzes stammt, und Erhitzen der verbleibenden Lösung auf eine Temperatur von 150 bis 250° C in einem Autoklaven unter ihrem autogenen Druck während einer Zeitdauer von 3 Stunden bis 10 Tagen zur Erzielung einer Suspension von Kristallen, hergestellt werden.

11. Verfahren nach Anspruch 10, worin die Reaktanten entsprechend der folgenden molaren Zusammensetzung vermischt werden:
- 0,1 bis 0,35 Tetrapropylammoniumhydroxid;
- SiO$_2$;

- 0,0025 bis 0,01 $Al_2O_3$;
- 0,2 bis 0,02 $B_2O_3$, und
- 20 bis 40 $H_2O$.

**12.** Verfahren nach Anspruch 5, worin in der zweiten Stufe zum Verbinden der Zeolithkristalle durch oligomeres Siliziumdioxid die Reaktanten genäß der folgenden Molzusammensetzung vermischt werden:
- 0,05 bis 0,15 Tetraalkylammoniumhydroxid;
- 4 bis 19 $SiO_2$ (aus dem Zeolith);
- 1 $SiO_2$ (aus dem Tetraalkylsilicat), und
- 80 bis 600 $H_2O$.

**13.** Verwendung des in Anspruch 1 definierten Katalysators zur selektiven Zersetzung von Cumolhydroperoxid zu Phenol und Aceton.

**14.** Verwendung des Katalysators nach Anspruch 13, worin Cumolhydroperoxid bei einer Temperatur von 20 bis 120° C, vorzugsweise von 40 bis 60° C mit dem Katalysator in Kontakt gebracht wird.